(19) Europäisches Patentamt
European Patent Office
Office européen des brevets

(11) EP 4 530 975 A1

(12) EUROPEAN PATENT APPLICATION

(43) Date of publication:
**02.04.2025 Bulletin 2025/14**

(21) Application number: **23465537.1**

(22) Date of filing: **26.09.2023**

(51) International Patent Classification (IPC):
***G06T 7/00*** $^{(2017.01)}$

(52) Cooperative Patent Classification (CPC):
**G06T 7/0012; G16H 30/40; G16H 50/20; G16H 50/30;** A61B 6/503; G06T 7/62; G06T 2207/10081; G06T 2207/30101

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(71) Applicant: **Siemens Healthineers AG
91301 Forchheim (DE)**

(72) Inventors:
- **TURCEA, Alexandru
105500 Busteni, Prahova (RO)**
- **CIMEN, Serkan
Jersey City, NJ, 07310 (US)**
- **Neumann, Dominik
91052 Erlangen (DE)**
- **BERGER, Martin
91088 Bubenreuth (DE)**
- **GULSUN, Mehmet Akif
Princeton, NJ, 08540 (US)**
- **ITU, Lucian Mihai
500294 Brasov (RO)**
- **SHARMA, Puneet
Princeton Junction, NJ, 08550 (US)**

(74) Representative: **Kraus & Lederer PartGmbB
Thomas-Wimmer-Ring 15
80539 München (DE)**

(54) **DETERMINATION OF HEMODYNAMIC INDICES**

(57) Techniques for processing multiple cardiac images are disclosed. The multiple cardiac images, each of which depicts a portion of coronary arteries, i.e., the same portion of coronary arteries, within an anatomical region of interest, are obtained (3100) either during or after an angiography exam. A respective set of lumen radius measurements is determined (3200) based on each of the multiple cardiac images and comprises multiple lumen radius measurements respectively associated with multiple locations of the portion of the coronary arteries. A maximum stenosis severity profile and a minimum stenosis severity profile associated with the portion of the coronary arteries are respectively determined (3300) based on the respective sets of lumen radius measurements. A lumen radius profile associated with the portion of the coronary arteries is determined (3400) based on the maximum stenosis severity profile and the minimum stenosis severity profile. A respective value for each of at least one hemodynamic index at a given location of the portion of the coronary arteries is determined (3500) based on the lumen radius profile.

FIG 3

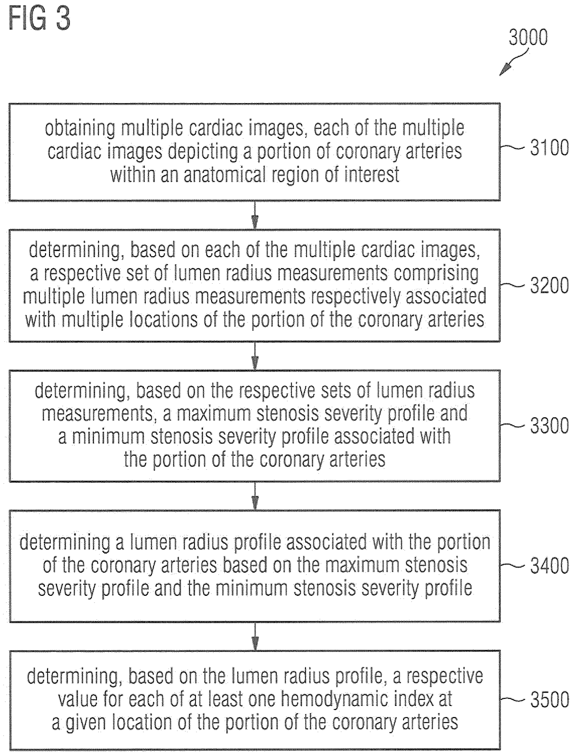


EP 4 530 975 A1

## Description

### TECHNICAL FIELD

Various examples of the disclosure generally relate to angiography. Various examples specifically relate to processing multiple cardiac images depicting a portion of coronary arteries within an anatomical region of interest to determine respective values of at least one hemodynamic index at a location of the portion of the coronary arteries.

### BACKGROUND

The gold standard for the functional assessment of coronary artery stenosis is the Fractional Flow Reserve (FFR) index. FFR is a hemodynamic index to determine the hemodynamic significance of a given epicardial coronary stenosis. It can be defined as the ratio of maximal achievable blood flow in a myocardial bed in the presence of an epicardial stenosis to the theoretical normal maximal flow in the same myocardial distribution. However, FFR is measured invasively using angiography. During such an invasive procedure, a contrast agent is injected through a catheter and into coronary arteries to make them easier to see. However, exposure to the contrast agent may cause kidney diseases or allergic reactions. Additionally, for an accurate measurement, FFR is measured when blood flow is at its highest, and thereby certain medicine, e.g., adenosine or papaverine is used to increase the blood flow. Such medicine may incur health risks.

To overcome drawbacks of invasive FFR measurements, technologies such as computational fluid dynamics (CFD) or machine learning (ML) can be used to derive a virtual FFR from X-ray Angiography or computed tomography.

For example, non-patent literature - Tröbs, Monique, et al. "Comparison of fractional flow reserve based on computational fluid dynamics modeling using coronary angiographic vessel morphology versus invasively measured fractional flow reserve." The American journal of cardiology 117.1 (2016): 29-35. - discloses a new approach to determine lesion-specific FFR on the basis of coronary anatomy as visualized by invasive coronary angiography.

As another example, non-patent literature - Itu, Lucian, et al. "A machine-learning approach for computation of fractional flow reserve from coronary computed tomography." Journal of applied physiology 121.1 (2016): 42-52. - discloses a machine-learning-based approach for computation of FFR index from coronary computed tomography.

Similarly, other hemodynamic indices like the resting distal coronary pressure to aortic pressure ratio (Pd/Pa), instantaneous wave-free ratio (iFR) , etc. may also be of interest and measured invasively or derived from either X-ray Angiography or computed tomography.

However, such methods for computing FFR or other hemodynamic indices focus on a region of interest that is visible in a single angiographic frame. Specifically, after acquiring one or more angiograms which comprise multiple temporal image/frame sequences per patient acquired under various angulations/views, each of the one or more angiograms need to be processed on a frame-by-frame (or image-by-image) basis to compute FFR or other hemodynamic indices.

An exemplary processing pipeline may comprise multiple image-based artificial intelligence (AI) modules and some or all of the AI modules may process each frame of the one or more angiograms but without taking (temporal) information between different frames, e.g., neighboring frames, due to various reasons such as the complexity of the task, non-availability of multi-frame training data, or other challenges.

For instance, a segmentation module/algorithm for extracting anatomical information provided as input for FFR computation from angiograms is typically applied on a single frame and processes the single frame without considering other frames. Even when the single frame is selected by either an experienced user or by an elaborate algorithm and taking factors like the presence and severity of foreshortening, vessel overlaps, image artifacts/quality, panning, vessel out-of-view, sub-optimal contrast agent, errors in results from other modules which are fed into this module, etc., the existing methods still cannot calculate or determine FFR or other hemodynamic indices precisely and reliably because they intrinsically do not consider lumen information from multiple time points of a cardiac cycle (or heartbeat, or heart cycle) in which the lumen diameter varies along the time points of the cardiac cycle.

### SUMMARY

Therefore, a need exists for advanced techniques for determining one or more hemodynamic indices such as FFR. Specifically, a need exists for advanced techniques of determining one or more hemodynamic indices for a portion of coronary arteries non-invasively. A need exists for precisely and reliably determining one or more hemodynamic indices for a specific patient.

This need is met by the features of the independent claims. The features of the dependent claims define embodiments.

A computer-implemented method for processing multiple cardiac images is provided. The method comprises obtaining multiple cardiac images, each of the multiple cardiac images depicting a portion of coronary arteries within an anatomical region of interest. The method further comprises determining, based on each of the multiple cardiac images, a respective set of lumen radius measurements comprising multiple lumen radius measurements respectively associated with multiple locations of the portion of the coronary arteries. The method also comprises determining, based on the respective sets of lumen radius measurements, a maximum stenosis severity profile and a minimum stenosis severity profile associated with the portion of the coronary arteries . The method still further comprises determining a lumen radius profile associated with the portion of the coronary arteries based on the maximum stenosis severity profile and the minimum stenosis severity profile and determining, based on the lumen radius profile, a respective value for each of at least one hemodynamic index at a given location of the portion of the coronary arteries.

According to various examples, the multiple cardiac images may comprise multiple frames of an angiogram which are acquired within a (single) cardiac cycle.

According to various examples, the at least one hemodynamic index may comprise at least one of a blood pressure, a blood flow rate, a fractional flow reserve, a heart rate, a cardiac index, a mean arterial pressure, a systemic vascular resistance index, a central venous pressure, and a central venous oxygen saturation.

A computing device comprising a processor and a memory is provided. Upon loading and executing program code from the memory, the processor is configured to perform a method for processing multiple cardiac images. The method comprises obtaining multiple cardiac images, each of the multiple cardiac images depicting a portion of coronary arteries within an anatomical region of interest. The method further comprises determining, based on each of the multiple cardiac images, a respective set of lumen radius measurements comprising multiple lumen radius measurements respectively associated with multiple locations of the portion of the coronary arteries . The method also comprises determining, based on the respective sets of lumen radius measurements, a maximum stenosis severity profile and a minimum stenosis severity profile associated with the portion of the coronary arteries. The method still further comprises determining a lumen radius profile associated with the portion of the coronary arteries based on the maximum stenosis severity profile and the minimum stenosis severity profile and determining, based on the lumen radius profile, a respective value for each of at least one hemodynamic index at a given location of the portion of the coronary arteries.

An angiography device comprising a computing device is provided. The computing device comprises a processor and a memory. Upon loading and executing program code from the memory, the processor is configured to perform a method for processing multiple cardiac images. The method comprises obtaining multiple cardiac images, each of the multiple cardiac images depicting a portion of coronary arteries within an anatomical region of interest. The method further comprises determining, based on each of the multiple cardiac images, a respective set of lumen radius measurements comprising multiple lumen radius measurements respectively associated with multiple locations of the portion of the coronary arteries. The method also comprises determining, based on the respective sets of lumen radius measurements, a maximum stenosis severity profile and a minimum stenosis severity profile associated with the portion of the coronary arteries. The method still further comprises determining a lumen radius profile associated with the portion of the coronary arteries based on the maximum stenosis severity profile and the minimum stenosis severity profile and determining, based on the lumen radius profile, a respective value for each of at least one hemodynamic index at a given location of the portion of the coronary arteries.

A computer program product or a computer program or a computer-readable storage medium including program code is provided. The program code can be executed by at least one processor. Executing the program code causes the at least one processor to perform a method for processing multiple cardiac images. The method comprises obtaining multiple cardiac images, each of the multiple cardiac images depicting a portion of coronary arteries within an anatomical region of interest. The method further comprises determining, based on each of the multiple cardiac images, a respective set of lumen radius measurements comprising multiple lumen radius measurements respectively associated with multiple locations of the portion of the coronary arteries. The method also comprises determining, based on the respective sets of lumen radius measurements, a maximum stenosis severity profile and a minimum stenosis severity profile associated with the portion of the coronary arteries. The method still further comprises determining a lumen radius profile associated with the portion of the coronary arteries based on the maximum stenosis severity profile and the minimum stenosis severity profile and determining, based on the lumen radius profile, a respective value for each of at least one hemodynamic index at a given location of the portion of the coronary arteries.

It is to be understood that the features mentioned above and those yet to be explained below may be used not only in the respective combinations indicated, but also in other combinations or in isolation without departing from the scope of the invention.

## BRIEF DESCRIPTION OF THE DRAWINGS

FIG. 1 schematically illustrates aspects with respect to a C-arm machine.

FIG. 2 schematically illustrates an exemplary 3D frontal view of a heart and of coronary arteries.

FIG. 3 is a flowchart of a method according to various examples.

FIG. 4 schematically illustrates multiple frames of an angiogram acquired at different time points.

FIG. 5 schematically illustrates one exemplary frame of an angiogram according to various examples.

FIG. 6 schematically illustrates various exemplary curves respectively depicting different sets of lumen radius measurements according to various examples.

FIG. 7 schematically illustrates various exemplary curves respectively depicting different aligned sets of lumen radius measurements according to various examples.

FIG. 8 schematically illustrates an exemplary maximum stenosis severity profile and an exemplary minimum stenosis severity profile according to various examples.

FIG. 9 is a block diagram of a computing device according to various examples.

## DETAILED DESCRIPTION OF THE DRAWINGS

Some examples of the present disclosure generally provide for a plurality of circuits or other electrical devices . All references to the circuits and other electrical devices and the functionality provided by each are not intended to be limited to encompassing only what is illustrated and described herein. While particular labels may be assigned to the various circuits or other electrical devices disclosed, such labels are not intended to limit the scope of operation for the circuits and the other electrical devices. Such circuits and other electrical devices may be combined with each other and/or separated in any manner based on the particular type of electrical implementation that is desired. It is recognized that any circuit or other electrical device disclosed herein may include any number of microcontrollers, a graphics processor unit (GPU), integrated circuits, memory devices (e.g., FLASH, random access memory (RAM), read only memory (ROM), electrically programmable read only memory (EPROM), electrically erasable programmable read only memory (EEPROM), or other suitable variants thereof), and software which co-act with one another to perform operation(s) disclosed herein. In addition, any one or more of the electrical devices may be configured to execute a program code that is embodied in a non-transitory computer readable medium programmed to perform any number of the functions as disclosed.

In the following, embodiments of the invention will be described in detail with reference to the accompanying drawings. It is to be understood that the following description of embodiments is not to be taken in a limiting sense. The scope of the invention is not intended to be limited by the embodiments described hereinafter or by the drawings, which are taken to be illustrative only.

The drawings are to be regarded as being schematic representations and elements illustrated in the drawings are not necessarily shown to scale. Rather, the various elements are represented such that their function and general purpose become apparent to a person skilled in the art. Any connection or coupling between functional blocks, devices, components, or other physical or functional units shown in the drawings or described herein may also be implemented by an indirect connection or coupling. A coupling between components may also be established over a wireless connection. Functional blocks may be implemented in hardware, firmware, software, or a combination thereof.

Hereinafter, techniques for determining a respective value of at least one hemodynamic index are described. The at least one hemodynamic index may comprise at least one of a blood pressure, a blood flow rate, a fractional flow reserve, a heart rate, a cardiac index, a mean arterial pressure, a systemic vascular resistance index, a central venous pressure, and a central venous oxygen saturation.

According to this disclosure, techniques for processing multiple cardiac images, each of which depicts the same portion of coronary arteries within an anatomical region of interest, are disclosed. For example, each of the multiple cardiac images may depict the same portion of either the left coronary artery or the right coronary artery of the same patient.

The multiple cardiac images may be obtained either during or after an angiography exam of the portion of coronary arteries within the anatomical region of interest. The processing may take place either during or after the angiography exam of the portion of coronary arteries, i.e., either real-time processing or post-processing is possible.

The multiple cardiac images, each of which depicts a portion of coronary arteries, i.e. , the same portion of coronary arteries, within an anatomical region of interest, are obtained either during or after an angiography exam. A respective set of lumen radius measurements is determined based on each of the multiple cardiac images and comprises multiple lumen radius measurements respectively associated with multiple locations of the portion of the coronary arteries. A maximum stenosis severity profile and a minimum stenosis severity profile associated with the portion of the coronary arteries are respectively determined based on the respective sets of lumen radius measurements. A lumen radius profile associated with the portion of the coronary arteries is determined based on the maximum stenosis severity profile and the minimum stenosis severity profile. A respective value for each of at least one hemodynamic index at a given location of the portion of the coronary arteries is determined based on the lumen radius profile.

According to various examples, the multiple cardiac images may be acquired at different time points of a cardiac cycle. For example, the multiple cardiac images may be acquired during a cardiac cycle of 1 second using a C-arm X-ray machine with a frame rate of 10. I.e., the multiple cardiac images encapsulate the lumen information of the portion of the coronary arteries from multiple time points of a single cardiac cycle, e.g. , changes in respective lumen diameter values at different locations of the portion of the coronary arteries.

By processing the multiple cardiac images using techniques disclosed herein, various hemodynamic indices can be determined or calculated in a cardiac-cycle-averaged manner. I.e., various hemodynamic indices can be precisely and reliably determined or calculated by anatomy and the flow throughout an entire cardiac cycle. For example, FFR can be determined or calculated as a ratio of mean distal to aortic coronary pressures over the entire cardiac cycle.

In general, the cardiac cycle is the performance of the human heart from the beginning of one heartbeat to the beginning of the next. It consists of two periods: one during which the heart muscle relaxes and refills with blood, called diastole, following a period of robust contraction and pumping of blood, called systole. After emptying, the heart relaxes and expands to receive another influx of blood returning from the lungs and other systems of the body, before again contracting to pump blood to the lungs and those systems. A normally performing heart must be fully expanded before it can efficiently pump again. Assuming a healthy heart and a typical rate of 70 to 75 beats per minute, each cardiac cycle, or heartbeat, takes about 0.8 second to complete the cycle.

Events and details of cardiac cycles can be illustrated using a Wiggers diagram. In the Wiggers diagram, the X-axis is used to plot time subdivided into the cardiac phases, while the Y-axis typically contains the following on a single grid: blood pressure, aortic pressure, ventricular pressure, atrial pressure, ventricular volume, and electrocardiogram. Accordingly, a specific cardiac cycle can be determined using electrocardiography.

According to various examples, the multiple cardiac images may be obtained directly from an angiography device or from a database for storing the multiple cardiac images acquired by the angiography device, e.g. , a picture archiving and communication system (PACS).

Generally, the typical maximum frame rates found on an angiography device such as a C-arm X-ray machine are 8, 15, or 30. This means that during a single cardiac cycle of 0.8 second, 6, 12, or 24 frames can be acquired by a C-arm X-ray machine.

According to various examples, the angiography device may comprise a C-arm X-ray machine, an intravascular ultrasound machine, an optical coherence tomography machine, or a coronary computed tomography angiography machine.

As an exemplary angiography device, FIG. 1 schematically illustrates aspects with respect to a C-arm machine 800. The C-arm machine 800 may be used for both angiography and fluoroscopy. In FIG. 1, the C-arm machine 800 is in neutral position $P_0$. It is possible to manipulate the C-arm machine 800 to be in a rotated position deviating from the neutral position $P_0$. The angle between the neutral position P0 and a specific rotated position is referred to as the angiography angle or the fluoroscopy angle. The C-arm machine 800 comprises a rotatable C arm 830 on which X-ray emission means 831 and X-ray detection means 832 may be mounted. The C arm 830 and thereby X-ray emission means 831 and X-ray detection means 832 are positioned to center around patient surface 840. X-ray emission means 831 may emit X-rays which may penetrate through a patient positioned on the patient surface 840. X-ray detection means 832 detects the X -rays emitted from X-ray emission means 831. When a patient on the patient surface 840 is injected with a radio-opaque contrast agent into the patient's vessels, some of the X-rays emitted by X-ray emission means 831 are absorbed by the radio-opaque contrast agent, leading X-ray detection means 832 to detect a sequence of images of the vessels filled with the radio-opaque contrast agent, i.e. an angiogram. X-ray emission means 831 and X-ray detection means 832 may also collectively be referred to as x-ray imaging means.

The C arm 830 may be coupled to a C arm rotation unit 820. The C arm rotation unit 820 may be any motorized means configured to rotate the C arm 830 according to an angiography angel or a fluoroscopy angle. The C arm rotation unit 820 may be attached to and controlled by a C arm control until 810. The C arm control unit 810 may be any kind of circuitry capable of controlling C arm 830. For example, the C arm control unit 810 may include a computing device.

The C-arm machine 800 may further include a control panel 850 mounted onto a side surface of patient surface support 841. The control panel 850 may be used to control C arm 830 in order to guide medical specialists to pathological vessels. Fig. 1 does not show any connections between control panel 850 and C arm 830 to simplify the depiction of the exemplary C-arm machine 800. In some examples, the connection may be wireless. In some further examples, the connection may be wired and may e.g. , be integrated into the ceiling of the room where the C-arm machine 800 is located.

The C-arm machine 800 may also include a display 860. The display 860 may be used to display information to the medical specialist, such as real-time fluoroscopy image with an overlaid vessel roadmap image including a path to one or more pathological vessels, and/or a location of the pressure wire for measuring the at least one measurement associated with the at least one hemodynamic index. Further, the display 860 may be used to display vessel segmentation data included in overlaid vessel roadmap images, including labels for various vessel segments. In some examples, display 860 may be a touch screen, which may be used to toggle the display of the vessel segmentation data on and off.

The C-arm machine 800 may be connectable to a database (not shown in FIG. 1), such as a PACS located within a local network of a hospital, for storing angiograms and/or measurements associated with the at least one hemodynamic index.

According to various examples, a name (e.g. , any one in the column "Abbreviation" in Table 1) of a specific segment of a coronary artery or of the entire coronary artery can be determined based on an anatomical region of interest of the angiography exam. For example, such an anatomical region of interest may comprise the left or right branch of the coronary vessel tree or a specific vessel section. The anatomical region of interest may be determined based on user input, or on a patient's electronic medical record included in a health information system. Additionally or optionally, the anatomical region of interest may be determined based on multiple predefined seed points.

For example, the portion of coronary arteries within the anatomical region of interest may be one or more segments of the coronary arteries of the heart. FIG. 2 schematically illustrates an exemplary 3D frontal view 200 of a heart and of coronary arteries. The segments of the coronary arteries in FIG. 2 are based on and numbered according to the vessel segmentation as proposed by the American Heart Association (AHA) and as amended by the Society of Cardiovascular Computed Tomography (SCCT). Table 1 provides a short description of the respective vessel segments as well as an abbreviation for each vessel segment.

*Table 1: Segments of the Coronary Arteries in FIG. 2.*

| Ref. Sign | Vessel Segment | Abbreviation | Description |
|---|---|---|---|
| 201 | proximal right coronary artery | pRCA | Ostium of the RCA to one-half the distance to the acute margin of heart |
| 202 | Mid RCA | mRCA | End of pRCA to the acute margin of heart |
| 203 | Distal RCA | dRCA | End of mRCA to origin of the PDA (posterior descending artery) |
| 204 | PDA-R | R-PDA | PDA from RCA |
| 205 | Left main | LM | Ostium of LM to bifurcation of LAD (left anterior descending artery) and LCx (left circumflex artery) |
| 206 | Proximal LAD | pLAD | End of LM to the first large septal or D1 (first diagonal) , whichever is most proximal |
| 207 | Mid LAD | mLAD | End of proximal LAD to one-half the distance to the apex |
| 208 | Distal LAD | dLAD | End of mid LAD to end of LAD |
| 209 | D1 | D1 | First diagonal branch D1 |
| 210 | D2 | D2 | Second diagonal branch D2 |
| 211 | Proximal LCx | pCx | End of LM to the origin of |
| | | | the OM1 (first obtuse marginal) |
| 212 | OM1 | OM1 | First OM1 traversing the lateral wall of the left ventricle |
| 213 | Mid and distal LCx | LCx | Traveling in the atrioventricular groove, distal to the OM1 branch to the end of the vessel or origin of the L-PDA |
| 214 | OM2 | OM2 | Second marginal OM2 |
| 215 | PDA-L | L-PDA | PDA from LCx |
| 216 | PLB-R | R-PLB | PLB from RCA |
| 217 | Ramus intermedius | RI | Vessel originating from the left main between the LAD and LCx in case of a trifurcation |
| 218 | PLB-L | L-PLB | PLB from LCx |

In addition to the vessel segments listed in Table 1, FIG. 2 also indicates the aortic valve 220.

FIG. 3 illustrates aspects with respect to processing multiple cardiac images, each of which depicts a portion of coronary arteries i.e., the same portion of coronary arteries, e.g., LAD (i.e. , a combination of pLAD 206, mLAD 207, and dLAD 208) , or RCA (i.e., a combination of pRCA 201, mRCA 202, and dRCA 203) as shown in FIG. 2, within an anatomical region of interest. The multiple cardiac images are obtained either during or after an angiography exam and thereby the processing can be either real-time processing or post-processing. A respective set of lumen radius measurements is determined based on each of the multiple cardiac images and comprises multiple lumen radius measurements respectively associated with multiple locations of the portion of the coronary arteries. A maximum stenosis severity profile and a minimum stenosis severity profile associated with the portion of the coronary arteries are respectively determined based on the respective sets of lumen radius measurements. A lumen radius profile associated with the portion of the coronary arteries is determined based on the maximum stenosis severity profile and the minimum stenosis severity profile. A respective value for each of at least one hemodynamic index at a given location of the portion of the coronary arteries is determined based on the lumen radius profile. Details of the method 3000 are described below.

Block 3100: obtaining multiple cardiac images, each of the multiple cardiac images depicting a portion of coronary arteries within an anatomical region of interest.

According to various examples, the multiple cardiac images may comprise cardiac images acquired under different acquisition angles.

For example, the multiple cardiac images may be obtained directly from an angiography device, e.g., the C-arm machine 800 of FIG. 1, under different acquisition angles. I.e. , the multiple cardiac images may respectively depict different views of the same portion of coronary arteries to increase accuracy and robustness of the determination of the radius measurements. For example, the true severity of a stenosis may need to be revealed by looking at it from multiple views, and multiple views can also help solve foreshortening-related issues.

Alternatively, the multiple cardiac images may be obtained from a database for storing the multiple cardiac images acquired by the angiography device, e.g., a PACS.

According to various examples, the multiple cardiac images may comprise cardiac images acquired using multi-phase coronary computed tomography angiography. I.e., the multiple cardiac images may be obtained from imaging data acquired using computed tomography angiography.

Hereinafter, techniques will be explained using frames of X-ray angiograms as an example of cardiac images. I.e. , a single cardiac image may correspond to a frame of an angiogram.

FIG. 4 schematically illustrates multiple frames t1-f10 of an angiogram acquired at different time points t1-t10 and each of the multiple frames f1-f10 depicts the same portion C of the coronary arteries within an anatomical region of interest.

For example, the multiple frames f1-f10 may be acquired during a single cardiac cycle of 1 second using a C-arm X-ray machine with a frame rate of 10. I.e., the multiple frames f1-f10 encapsulate the lumen information of the portion C of the coronary arteries from multiple time points of a single cardiac cycle, e.g., changes in respective lumen diameter values at different locations of the portion C of the coronary arteries.

To improve the accuracy of determination of respective values of the at least one hemodynamic index at a (given) position, it is possible to automatically and precisely select or determine, among frames of an angiogram, multiple appropriate frames, e.g., the multiple frames f1-f10, where the entire portion C of the coronary arteries is visible.

According to various examples, said obtaining of the multiple cardiac images may comprise obtaining an angiogram acquired during an angiography exam of the anatomical region of interest and selecting, based on at least one pre-defined criterion, the multiple cardiac images among frames of the angiogram.

Such pre-defined criterion may comprise at least one of the following:

- the portion of coronary arteries is well visible;
- minimal foreshortening appears;
- no or minimal vessel overlaps appear;
- image quality, especially around the lumen borders, is good, e.g., above a threshold;
- no artifact presents; and
- the entire portion of coronary arteries is in the field of view.

For example, the multiple cardiac images may be selected from frames acquired in a single cardiac cycle, e.g., selecting 5 frames from the 10 frames f1-f10 acquired in a single cardiac cycle.

Alternatively or optionally, the multiple cardiac images may be selected from frames acquired in different cardiac cycles. For example, an acquired angiogram may comprise 100 frames acquired in 10 cardiac cycles, e.g., first to tenth cardiac cycles, and 10 frames, e.g., first to tenth frames, are acquired in each cardiac cycle. A first cardiac image may be selected from respective first frames acquired in respective cardiac cycles. A further or second cardiac image may be selected from respective second (or any one of the third to tenth) frames acquired in respective cardiac cycles.

As a further example, 10 cardiac images may be selected from frames acquired in 10 different cardiac cycles. Each one of the 10 cardiac images may be selected from respective frames acquired at the same time point within respective cardiac cycles. E.g., the first cardiac image, e.g., f1, among the 10 cardiac images may be selected from all the first frames respectively acquired in the first to tenth cardiac cycles; the second cardiac image, e.g., f2, among the 10 cardiac images may be selected from all the second frames respectively acquired in the first to tenth cardiac cycles; and so on.

According to various examples, after obtaining the multiple cardiac images, the method 3000 may further comprise determining the anatomical region of interest based on multiple predefined seed points.

For example, proximal seed points may comprise the catheter tip, and distal seed points may comprise one or more points selected on the main vessels or on side branches with significant stenoses .

Optionally, the portion of coronary arteries may be determined based on angulation information for acquiring the multiple cardiac images.

Optionally or additionally, the method 3000 may further comprise registering the multiple cardiac images.

For example, the proximal and distal seed points may be co-registered across the multiple cardiac images to ensure that they represent the same anatomical region of interest.

According to various examples, said registration or co-registration may comprise applying a diffeomorphic registration algorithm, e.g., as disclosed in a non-patent literature - Ashburner, John. "A fast diffeomorphic image registration algorithm. " Neuroimage 38.1 (2007) : 95-113., on the vesselness maps extracted from each frame; using the obtained deformation fields to track each of the seed points from each frame onto all of the other frames; and aggregating the tracked seed points via a density-based clustering algorithm such as DB Scan (i.e., Density-based spatial clustering of applications with noise) , e.g., as disclosed in a non-patent literature - Hu, Lihua, et al. "KR-DBSCAN: A density-based clustering algorithm based on reverse nearest neighbor and influence space." Expert Systems with Applications 186 (2021): 115763.

According to various examples, the method 3000 may further comprise segmenting the anatomical region of interest across the multiple cardiac images, i.e., separating the anatomical region of interest from the background in each of the multiple cardiac images.

Block 3200: determining, based on each of the multiple cardiac images, a respective set of lumen radius measurements comprising multiple lumen radius measurements respectively associated with multiple locations of the portion of the coronary arteries.

According to various examples, the lumen radius measurements may be determined or calculated using any one of the existing techniques or algorithms related to quantitative coronary angiography. For example, non-patent literature - Garrone, Paolo, et al. "Quantitative coronary angiography in the current era: principles and applications." Journal of interventional cardiology 22.6 (2009): 527-536. - provides a comprehensive and updated viewpoint on quantitative coronary angiography. It is also conceivable that various existing machine learning algorithms or image processing techniques can be used to determine the lumen radius measurements.

FIG. 5 schematically illustrates one exemplary frame 1000 of an angiogram according to various examples. The frame 1000 may depict the portion C of coronary arteries as shown in FIG. 4. A centerline 1100 is generated between the starting point 1101 and the ending point 1124 of the portion C of coronary arteries. Between the starting point 1101 and the ending point 1124, there are multiple location points 1102-1123. For each of the location points 1101-1124, a respective lumen radius associated with such a location point may be measured or determined, and thereby a set of lumen radius measurements comprising the respective lumen radius at each of the location points 1101-1124 can be determined or calculated.

For example, at location point 1107, two lumen radii r1 and r2 may be determined or measured. The respective lumen radius associated with location point 1107 may be determined as an average of r1 and r2. It is also possible to determine or select the lumen radius associated with location point 1107 as either r1 or r2. Optionally, r1 and r2 may be determined based on different frames acquired using different acquisition angles.

According to various examples, for each of the location points 1101-1124, it is also possible to determine multiple, e.g., 3, 4, 8, or even more, lumen radii based on multiple frames acquired using different acquisition angles and determine an average of the multiple lumen radii as the respective lumen radius for the respective set of lumen radius measurements.

Similarly, for each one of the multiple frames f1-f10 of FIG. 4, a respective set of lumen radius measurements comprising the respective lumen radius at each of the location points 1101-1124 can be determined or calculated. I.e., ten sets s1-s10 of lumen radius measurements can be determined respectively for the ten frames f1-f10, and each of the ten sets s1-s10 comprises lumen radius measurements respectively associated with the location points 1101-1124.

To facilitate the determination of the respective sets s1-s10 of the lumen radius measurements, a segmentation algorithm may be applied to the ten frames f1-f10, respectively, to separate the portion C of the coronary arteries from the background of the respective frame.

According to various examples, said determining of the respective set of lumen radius measurements may comprise segmenting the portion C of the coronary arteries from the respective cardiac image of the multiple cardiac images f1-t10 and determining the respective set s1-s10 of the lumen radius measurements based on the respective segmented portion of the coronary arteries.

According to various examples, after segmenting the portion C of the coronary arteries from the respective cardiac image of the multiple cardiac images f1-f10, the respective segmented portion of the coronary arteries may be co-registered along the centerline 1100 and/or based on landmarks detected in the respective frames f1-f10, e.g., bifurcations, stenoses, calcifications, and etc.

Block 3300: determining, based on the respective sets of lumen radius measurements, a maximum stenosis severity profile and a minimum stenosis severity profile associated with the portion of the coronary arteries.

According to various examples, the maximum stenosis severity profile and the minimum stenosis severity profile may be respectively determined based on local concavities and/or local convexities of multiple curves and each of the multiple curves may depict a respective relationship between a respective set of the lumen radius measurements and the multiple locations of the portion of the coronary arteries.

For example, as shown in FIG. 6, after determining the respective sets sl-s10 of the lumen radius measurements, respective curves (or original radius profiles) p1-p10 may be determined or plotted for each of the sets s1-s10 using interpolation such as linear interpolation, polynomial interpolation, spline interpolation, and/or mimetic interpolation. Optionally, the sets s1-s10 may be determined based on frames f1-f10 acquired in a single cardiac cycle or heart cycle. The x-axis of each curve may indicate respective distances (or 2D projection distances) between the catheter tip and each of the location points 1101-1124, and the y-axis may indicate respective lumen radii associated with the respective location points. Then, the maximum stenosis severity profile and the minimum stenosis severity profile may be respectively determined based on local concavities or convexities of the respective curves p1-p10. For example, the second derivatives at each of the location points 1101-1124 may be calculated for each of the respective curves p1-p10.

At a location point of a curve, if the second derivative is positive the curve is concave up (also referred to as convex) at such a location point, meaning that the tangent line at such a location point lies below the curve; similarly, if the second derivative is negative the curve is concave down (also simply called concave) at such a location point, meaning that the tangent line at such a location point lies above the curve; if the second derivative changes sign, the curve will switch from concave down to concave up, or vice versa, and a point where this occurs is called an inflection point.

According to various examples, the second derivatives may be computed via central finite differences.

In practice, due to vessel deformations caused by heart motion, the respective sets s1-s10 of the lumen radius measurements (or the radius profiles/curves p1-p10) may be not aligned. To more precisely determine the respective values of the at least one hemodynamic indices, the respective sets s1-s10 of the lumen radius measurements (or the radius profiles/curves p1-p10) may be warped before determining the maximum stenosis severity profile and the minimum stenosis severity profile.

For example, a dynamic time warping algorithm, e.g. , as disclosed in a non-patent literature - Salvador, Stan, and Philip Chan. "Toward accurate dynamic time warping in linear time and space. " Intelligent Data Analysis 11.5 (2007) : 561-580. - may be used for warping the respective sets sl-s10 of the lumen radius measurements. Alternatively, it is also possible to use an optimal subsequence bijection algorithm such as that disclosed in a non-patent literature - Latecki, Longin Jan, et al. "Optimal subsequence bijection. " Seventh IEEE International Conference on Data Mining (ICDM 2007). IEEE, 2007.

Optionally or additionally, said determining of the maximum stenosis severity profile and the minimum stenosis severity profile may comprise determining a reference set of the lumen radius measurements, aligning each of the respective sets of the lumen radius measurements with the reference set of the lumen radius measurements, and determining the maximum stenosis severity profile and the minimum stenosis severity profile based on the respective aligned sets of the lumen radius measurements.

For example, the reference set of the lumen radius measurements may be randomly or arbitrarily selected from the ten sets s1-s10 of the lumen radius measurements. For instance, s1 is selected as the reference set of the lumen radius measurements and the other sets s2-s10 can be respectively aligned with the reference set s1 using dynamic time warping algorithm, e.g., the dtw function of Matlab, i.e., executing dtw (s1, s2) , dtw (s1, s3) , dtw (sl,s4), ..., dtw (s1,s10).

According to various examples, said determining of the reference set of the lumen radius measurements may comprise selecting the set of the lumen radius measurements having the longest length as the reference set of the lumen radius measurements. Optionally or additionally, the set of the lumen radius measurements having the longest length may be determined by sorting the respective sets of the lumen radius measurements based on respective lengths of the respective sets in descending or ascending order.

For example, each of the ten sets s1-s10 of the lumen radius measurements may be indicated as a row or column vector, and the length of a respective set corresponds to the number of elements or entries of the respective vector.

According to various examples, said aligning of each of the respective sets of the lumen radius measurements with the reference set of the lumen radius measurements may comprise, for each of the respective unaligned sets of the lumen radius measurements, iteratively performing the following steps:

- selecting the longest set of the lumen radius measurements among all the unaligned sets of the lumen radius measurements;

- aligning the selected set of the lumen radius measurements with the reference set of the lumen radius measurements; and
- updating the reference set of the lumen radius measurements based on all aligned sets of the lumen radius measurements.

For example, after sorting the respective sets s1-s10 of the lumen radius measurements based on respective lengths of the respective sets s1-s10 in descending order, the first sorted set, i.e., the longest set, e.g., s5, may be selected as the initial reference set and then the second longest set, e.g. , s2, may be aligned with the initial reference set s5 to obtain the aligned set s2' of s2. Next, the reference set is updated based on the initial reference set s5 and the aligned set s2' of s2. For example, the reference set may be determined or calculated based on either an element-wise average or an element-wise weighted average of s5 and s2'. Similarly, the third longest set may be aligned with the updated reference set and then the reference set may be updated again by further considering the aligned set of the third longest set. I.e., said aligning and updating may be iteratively performed until the shortest set has been aligned.

Optionally or additionally, said aligning may be based on one or more anatomical landmarks within the anatomical region of interest.

For example, the one or more anatomical landmarks may be associated with the portion of coronary arteries, e.g., bifurcations, stenoses, calcifications, and etc.

FIG. 7 schematically illustrates various exemplary curves respectively depicting different aligned sets of lumen radius measurements according to various examples. For example, FIG. 7 may schematically illustrate respective aligned/warped curves p1'-p10' of the various exemplary curves p1-p10 shown in FIG. 6. I.e., the respective aligned curves p1'-p10' shown in FIG. 7 can be determined or plotted for each of the aligned sets s1'-s10' of s1-s10 using interpolation. The curves p1'-p10' shown in FIG. 7 may comprise warped radius profiles extracted from one cardiac/heart cycle.

According to various examples, the maximum stenosis severity profile and the minimum stenosis severity profile may be respectively determined based on local concavities and/or local convexities of the multiple aligned curves p1'-p10'.

In general, coronary stenosis refers to an abnormally narrowed coronary artery. The stenosis is usually discrete, but may sometimes be diffuse and involve a long segment of an artery. In coronaries, stenoses are usually located in the large epicardial arteries with diameters of more than 2 mm, but they may also occur in smaller arteries. In the majority of cases, a stenosis is caused by atherosclerosis, but other causes such as systemic inflammatory diseases may be involved. Coronary stenoses are usually chronic fixed changes, but sometimes a transient, dynamic stenosis due to spasm of the artery may happen.

From a pathophysiological point of view, a coronary stenosis impairs flow to the subtending myocardium, as the perfusion pressure is decreased distal to the stenosis. The flow dynamics in a vessel is governed by Poiseuille's law, which states that resistance in a vessel has an inverse relationship with the radius of the vessel to the fourth power. For example, the diameter (and therefore the radius as well) of a coronary artery may be reduced by two-thirds (for example, an artery with a diameter of 3 mm with a fixed stenosis at a discrete segment may have a residual diameter of only 1 mm in that segment) . In that case, the remaining diameter (and the radius) in the diseased segment will be one-third of the initial diameter/radius, and according to Poiseuille's law, the resistance within that stenotic segment will increase by 81-fold. If the perfusion pressure in that coronary artery remained constant, then the blood flow distal to this stenosis would decrease by 81-fold.

Based on these intrinsic characteristics of coronary artery stenoses, individual stenosis can be determined by a significant reduction followed by a significant increase in the lumen radius value or profiles. An individual stenosis generally starts at a location with a (local) maximum radius and ends at a location with a further (local) maximum radius. Between the two local maximum radius locations, there is a significant reduction in the radius value, i.e., the segment of the lumen radius profile between the two local maximum radius locations looks like a convex curve. FIG. 8 schematically illustrates an exemplary maximum stenosis severity profile 2100 and an exemplary minimum stenosis severity profile 2200 according to various examples. The maximum stenosis severity profile 2100 and the minimum severity profile 2200 may be determined according to the following steps:

Step 1: determining, based on the aligned curves p1'-p10', one or more local maximum lumen radius of the portion C of the coronary arteries and respective locations of the portion C of the coronary arteries with which the one or more local maximum lumen radius are associated; and determining, based on the aligned curves p1'-p10', respective minimum lumen radii associated with the respective locations with which the one or more local maximum lumen radius are associated.

For example, points M1 M2, M3, and M4 respectively indicate four local maximum lumen radii. The points M1, M2, M3, and M4 are respectively associated with locations 11, 12, 13, and 14 of the portion C of the coronary arteries. After determining the locations 11, 12, 13, and 14, the respective minimum lumen radii associated with the four locations 11, 12, 13, and 14 can be determined, i.e., the respective radii indicated by points A1, A2, A3, and A4.

According to this disclosure, the maximum and minimum lumen radii associated with the same location of the portion C of the coronary arteries may be determined among different frames, e.g. , f1-f10, of an angiogram.

Step 2: determining stenoses based on the local maximum lumen radii.

As described above, an individual stenosis generally starts at a location with a (local) maximum radius and ends at a location with a further (local) maximum radius. As shown in FIG. 8, the three segments of curves, e.g., M1M2, M2M3, andM3M4, in between respective two neighboring points of the four points M1, M2, M3, and M4 indicate three stenoses S1 (i .e. , between locations 11 and 12), S2 (i. e. , between locations 12 and 13), and S3 (i. e. , between locations 13 and 14), respectively.

Step 3: determining, based on the aligned curves p1'-p10', a respective local minimum lumen radius for each of the determined stenoses, e.g., S1, S2 and S3, and respective locations of the portion C of the coronary arteries with which the respective local minimum lumen radii are associated; and determining, based on the aligned curves p1'-p10', respective maximum lumen radii associated with the respective locations with which the respective local minimum lumen radii are associated.

As shown in FIG. 8, points N1, N2, and N3 respectively indicate the minimum lumen radii of the three stenoses S1, S2, and S3. The points N1, N2, and N3 are respectively associated with locations 15, 16, and 17 of the portion C of the coronary arteries. After determining the locations 15, 16, and 17, the respective maximum lumen radii associated with the three locations 15, 16, and 17 can be determined, i.e. , the respective radii indicated by points B1, B2, and B3.

According to this disclosure, the maximum and minimum lumen radii associated with the same location of the portion C of the coronary arteries may be determined among different frames, e. g. , f1-f10, of an angiogram.

Step 4: for each of the determined stenoses, e.g., S1, S2, and S3, determining respective segments of both the maximum severity profile 2100 and the minimum severity profile by interpolating lumen radius values segment-wise from the start to the end of the respective stenosis.

In general, for a specific stenosis such as any one of S1, S2, and S3, the respective segment of the maximum severity profile 2100 has, compared to the respective segment of the minimum severity profile 2200, a larger lumen radius at the start and end of the stenosis, respectively, and on the other hand, has a smaller radius at the location of the minimal lumen radius. This means that the maximum and minimum severity profiles 2100 and 2200 typically intersect with each other within one stenosis.

For example, for stenosis S1, the respective segment of the maximum severity profile 2100 may start from point M1, via point N1, and end at point M2 and the respective segment of the minimum severity profile 2200 may start from point A1, via point B1, and end at point A2; for stenosis S2, the respective segment of the maximum severity profile 2100 may start from point M2, via point N2, and end at point M3 and the respective segment of the minimum severity profile 2200 may start from point A2, via point B2, and end at point A3; for stenosis S3, the respective segment of the maximum severity profile 2100 may start from point M3, via point N3, and end at point M4 and the respective segment of the minimum severity profile 2200 may start from point A3, via point B3, and end at point A4.

According to various examples, for an individual stenosis determined in step 2 explained above, multiple segments of the stenosis may be determined based on one or more local maximum lumen radii between the start and end of the stenosis. For each segment, respective segments of the maximum and minimum severity profiles 2100 and 2200 can be determined or revised more precisely by performing the steps 1-4.

Additionally, or optionally, each segment may be divided into multiple sub-segments based on one or more local maximum lumen radii between the start and end of the respective segment. For each sub-segment, the maximum and minimum severity profiles 2100 and 2200 can be determined or revised more precisely by performing the steps 1-4, too.

In summary, steps 1-4 can be applied to different segments of the portion C of the coronary arteries and thereby the maximum and minimum severity profiles 2100 and 2200 can be determined. The more segments the more precise the two profiles 2100 and 2200. The techniques disclosed herein can ensure that different local severity values are obtained for each stenosis, while at the same time, the radius profile displays no discontinuities.

Block 3400: determining a lumen radius profile associated with the portion of the coronary arteries based on the maximum stenosis severity profile and the minimum stenosis severity profile.

For example, the lumen radius profile associated with the portion C of the coronary arteries may be determined by randomly or arbitrarily selecting a curve between the maximum stenosis severity profile 2100 and the minimum stenosis severity profile 2200. I.e. , at each of the location points 1101-1124, a respective lumen radius value may be randomly or arbitrarily selected such that the selected value is between the corresponding values of the maximum stenosis severity profile 2100 and the minimum stenosis severity profile 2200.

For example, the lumen radius profile may be determined according to the following equation:

$$\vec{R} = severity * \overrightarrow{Max} + (1 - severity) * \overrightarrow{Min} \qquad (1)$$

in which, $\vec{R}$, $\overrightarrow{\mathrm{Max}}$ , and $\overrightarrow{\mathrm{Min}}$ are vectors respectively indicating the lumen radius profile, the maximum stenosis severity profile 2100, and the minimum stenosis severity profile 2200. *severity* is a scalar ranging from 0 to 1. I.e., the lumen radius profile can be determined based on a convex linear combination of the maximum stenosis severity profile 2100 and the minimum stenosis severity profile 2200, and the lumen radius profile may vary as the value of *severity* varies.

In general, it is possible to determine the lumen radius profile, e.g., one or more desired lumen radius profiles, based on any combination of the maximum stenosis severity profile 2100 and the minimum stenosis severity profile 2200, e.g., non-linear combination.

According to various examples, said determining of the lumen radius profile associated with the portion of the coronary arteries may be further based on one or more (given) stenosis severity values and each of the one or more stenosis severity values is associated with a respective segment of the portion of the coronary arteries.

For example, the three different artery segments 2110, 2120, and 2130 shown in FIG. 8 may be associated with three different severity values and the other segments of the portion C of the coronary arteries may share the same severity values. The lumen radius profile may be determined using equation (1) based on all these four severity values associated with different segments of the portion C of the coronary arteries.

Optionally or additionally, the method 3000 may further comprise selecting each of the one or more given stenosis severity values between the minimum and maximum stenosis severity values associated with the respective segment of the portion of the coronary arteries.

Block 3500: determining, based on the lumen radius profile, a respective value for each of at least one hemodynamic index at a given location of the portion of the coronary arteries.

For example, a respective lumen radius value can be determined based on the lumen radius profile for each of the location points 1101-1124. Then, a respective value for each of at least one hemodynamic index, e.g. , FFR, at a given location, e.g. , location point 115, of the portion C of the coronary arteries can be determined or calculated using any one of the existing CFD-based or ML-based methods, e.g., an FFR solver.

According to various examples, the method 3000 may further comprise determining one or more further lumen radius profiles associated with the portion of the coronary arteries based on the maximum stenosis severity profile and the minimum stenosis severity profile. For each of the at least one hemodynamic index, the method 3000 may still comprise determining, based on each of the one or more further lumen radius profiles, a further respective value at the given location of the portion of the coronary arteries, and determining, based on the respective value and the further respective values, an uncertainty range associated with the respective hemodynamic index.

For example, five lumen radius profiles associated with the portion of the coronary arteries may be determined based on the maximum stenosis severity profile 2100 and the minimum stenosis severity profile 2200, and then five FFR values can be determined, using an FFR solver, for the same location of the portion of the coronary arteries respectively based on the five lumen radius profiles. Next, an uncertainty range associated with the FFR can be determined based on the five FFR values using various existing uncertainty quantification methods such as LHS (Latin Hypercube Sampling), Reliability Methods, Stochastic Expansion Methods, Adaptive Sampling, etc.

According to various examples, techniques disclosed and/or cited in a non-patent literature - Sankaran, Sethuraman, et al. "Uncertainty quantification in coronary blood flow simulations: impact of geometry, boundary conditions and blood viscosity." Journal of biomechanics 49.12 (2016): 2540-2547. - may be employed herein.

One important step for these existing uncertainty quantification methods may be to define the input and output variables of interest:

**Input variables:** These reflect the radius values. Each location could represent an independent input variable however, it may be suboptimal to handle each location independently because the radius values at neighboring locations are correlated with each other. Hence, one could define an input variable as a percentage value between the minimal and maximal radius values. This percentage value could be associated with a vessel segment, where the same value of the input variable may be employed. Alternatively, one could have a percentage value, i.e., input variable, for each stenosis, for healthy segments, main/side branch segments, etc. A more fine-grained approach (many input variables) or a more coarse-grained approach (few input variables) may be chosen. In general, the higher the number of input variables, the longer the runtime for computing FFR with uncertainty.

**Output variables:** FFR values and other hemodynamic indices at different key locations in a region of interest. The runtime is influenced minimally by the number of output variables, hence, a trade-off is not required.

According to this disclosure, the multiple cardiac images may comprise cardiac images acquired under different acquisition angles. If the correspondence between certain landmarks on the target coronary vessel, e.g. , the portion C of coronary arteries, is given in the multiple cardiac images or views via user input or an adequate multi-view vessel matching algorithm, the techniques described herein may further take into account the radius profiles from multiple views, where each view contributes one or more frames to the co-registration/registration procedure. More comprehensive co-registration methodologies may be employed to compensate for e.g., different lengths of the target vessel (due to projection geometry).

FIG. 9 is a block diagram of a computing device 9000 according to various examples. The computing device 9000 may comprise a processor 9020, a memory 9030, and an input/output interface 9010. The processor 9020 is configured to load program code from the memory 9030 and execute the program code. Upon executing the program code, the processor 9020 performs the method 3000 for processing multiple cardiac images depicting the same coronary artery of interest, e.g., the portion C of coronary arteries within an anatomical region of interest.

The computing device 9000 may be embedded in or connected with an angiography device such as the C-arm machine 800. I.e., the angiography device comprising the computing device 9000 may be configured to perform the method 3000.

Referring to FIG. 1 again, the C-arm machine 800 may further comprise the computing device 9000 configured to perform the method 3000. The computing device 9000 may be the C arm control unit 810 and/or the control panel 850. I.e. , the computing device 9000 may be embedded in or connected with the C-arm machine 800, and thereby the C-arm machine 800 may be also configured to perform the method 3000.

Summarizing, techniques have been described that facilitate determining at least one hemodynamic index by taking lumen information from multiple time points of a cardiac cycle into account, e.g., in a cardiac-cycle-averaged manner. The techniques allow for a more robust assessment and for the estimation of an uncertainty range in the respective computed hemodynamic index, e.g., FFR. Moreover, such techniques may also increase the acceptability of such a solution among clinicians, since visually they also assess an angiographic view dynamically, i.e., by analyzing the clip while being played in a continuous loop.

According to this disclosure, various hemodynamic indices can be precisely and reliably determined or calculated by anatomy and the flow throughout an entire cardiac cycle. For example, FFR can be determined or calculated as a ratio of mean distal to aortic coronary pressures over an entire cardiac cycle.

Although the disclosure has been shown and described with respect to certain preferred embodiments, equivalents and modifications will occur to others skilled in the art upon the reading and understanding of the specification. The present disclosure includes all such equivalents and modifications and is limited only by the scope of the appended claims.

For illustration, the disclosure is explained in detail based on X-ray angiography, the techniques disclosed herein can be also applied to angiography that may be done using scans instead of X-rays, such as CT (computed tomography) angiography or MRI (Magnetic resonance imaging) angiography.

Further, the approaches described herein may be used also when certain parts of the region of interest are not well visible on a certain frame, e.g., due to foreshortening, vessel overlap, etc.

In addition, the approaches described herein may be used also when the multiple cardiac images are obtained from different angiography views / acquired under different acquisition angles. Certain regions of interest may be recommended depending on the angulation of the acquisition (e.g. LM/pLAD/proximal LCx for the spider view). By combining the recommended regions of interest from the various angulations, a more accurate representation of the entire region of interest may be obtained.

Further, the approaches described herein may be used also when the multiple cardiac images are obtained from different imaging modalities: OCT / IVUS: highly accurate anatomical information on a single vessel/vessel segment or CCTA: entire coronary arterial tree modeled in 3D.

## Claims

1. A computer-implemented method (3000), comprising:

    - obtaining (3100) multiple cardiac images (f1-f10), each of the multiple cardiac images (f1-f10) depicting a portion (C) of coronary arteries within an anatomical region of interest
    - determining (3200), based on each of the multiple cardiac images (f1-f10), a respective set of lumen radius measurements comprising multiple lumen radius measurements respectively associated with multiple locations (1101-1124) of the portion (C) of the coronary arteries;
    - determining (3300), based on the respective sets of lumen radius measurements, a maximum stenosis severity profile (2100) and a minimum stenosis severity profile (2200) associated with the portion (C) of the coronary arteries;
    - determining (3400) a lumen radius profile associated with the portion (C) of the coronary arteries based on the maximum stenosis severity profile (2100) and the minimum stenosis severity profile (2200); and
    - determining (3500), based on the lumen radius profile, a respective value for each of at least one hemodynamic index at a given location of the portion (C) of the coronary arteries.

2. The computer-implemented method (3000) of claim 1, said determining (3300) of the maximum stenosis severity profile (2100) and the minimum stenosis severity profile (2200) comprising:

- determining a reference set of the lumen radius measurements;
- aligning each of the respective sets (p1-p10) of the lumen radius measurements with the reference set of the lumen radius measurements;
- determining the maximum stenosis severity profile (2100) and the minimum stenosis severity profile (2200) based on the respective aligned sets (p1'-p10') of the lumen radius measurements.

**3.** The computer-implemented method (3000) of claim 2, said determining of the reference set of the lumen radius measurements comprising:

- selecting the set of the lumen radius measurements having the longest length as the reference set of the lumen radius measurements.

**4.** The computer-implemented method (3000) of claim 3, said aligning of each of the respective sets (p1-p10) of the lumen radius measurements with the reference set of the lumen radius measurements comprising:
for each of the respective unaligned sets of the lumen radius measurements, iteratively performing the following steps:

- selecting the longest set of the lumen radius measurements among all the unaligned sets of the lumen radius measurements;
- aligning the selected set of the lumen radius measurements with the reference set of the lumen radius measurements;
- updating the reference set of the lumen radius measurements based on all aligned sets of the lumen radius measurements.

**5.** The computer-implemented method (3000) of any one of claims 2-4,
wherein said aligning is based on one or more anatomical landmarks within the anatomical region of interest.

**6.** The computer-implemented method (3000) of any one of the preceding claims,
the maximum stenosis severity profile (2100) and the minimum stenosis severity profile (2200) are respectively determined based on local concavities of multiple curves, each of the multiple curves depicting a respective relationship between a respective set of the lumen radius measurements and the multiple locations (1101-1124) of the portion (C) of the coronary arteries.

**7.** The computer-implemented method (3000) of any one of the preceding claims, said obtaining (3100) of the multiple cardiac images (f1-f10) comprising:

- obtaining an angiogram acquired during an angiography exam of the anatomical region of interest;
- selecting, based on at least one pre-defined criterion, the multiple cardiac images (f1-f10) among frames of the angiogram.

**8.** The computer-implemented method (3000) of any one of the preceding claims, said determining (3200) of the respective set of lumen radius measurements comprising:

- segmenting the portion (C) of the coronary arteries from the respective cardiac image of the multiple cardiac images (f1-f10); and,
- determining the respective set of the lumen radius measurements based on the respective segmented portion of the coronary arteries.

**9.** The computer-implemented method (3000) of any one of the preceding claims, further comprising:

- determining the anatomical region of interest based on multiple predefined seed points.

**10.** The computer-implemented method (3000) of any one of the preceding claims, further comprising:

- registering the multiple cardiac images.

**11.** The computer-implemented method (3000) of any one of the preceding claims,
wherein said determining of the lumen radius profile associated with the portion of the coronary arteries is further based on one or more given stenosis severity values and each of the one or more stenosis severity values is associated with a respective segment of the portion of the coronary arteries.

**12.** The computer-implemented method (3000) of claim 11, further comprising:

- selecting each of the one or more given stenosis severity values between the minimum and maximum stenosis severity values associated with the respective segment of the portion of the coronary arteries.

**13.** The computer-implemented method (3000) of any one of the preceding claims, further comprising:

- determining one or more further lumen radius profiles associated with the portion of the coronary arteries based on the maximum stenosis severity profile (2100) and the minimum stenosis severity profile (2200);

for each of the at least one hemodynamic index:

- determining, based on each of the one or more further lumen radius profiles, a further respective value at the given location of the portion of the coronary arteries;
- determining, based on the respective value and the further respective values, an uncertainty range associated with the respective hemodynamic index.

**14.** The computer-implemented method (3000) of any one of the preceding claims, wherein the multiple cardiac images comprise cardiac images acquired under different acquisition angles.

**15.** The computer-implemented method (3000) of any one of the preceding claims, wherein the multiple cardiac images comprise cardiac images acquired using multi-phase coronary computed tomography angiography.

**16.** A computing device (9000), the device comprising a processor (9020) and a memory (9030), wherein upon loading and executing program code from the memory (9030), the processor (9020) is configured to perform the method (3000) of any one of the preceding claims.

**17.** An angiography device (800) comprising the computing device (9000) of claim 16.

FIG 1

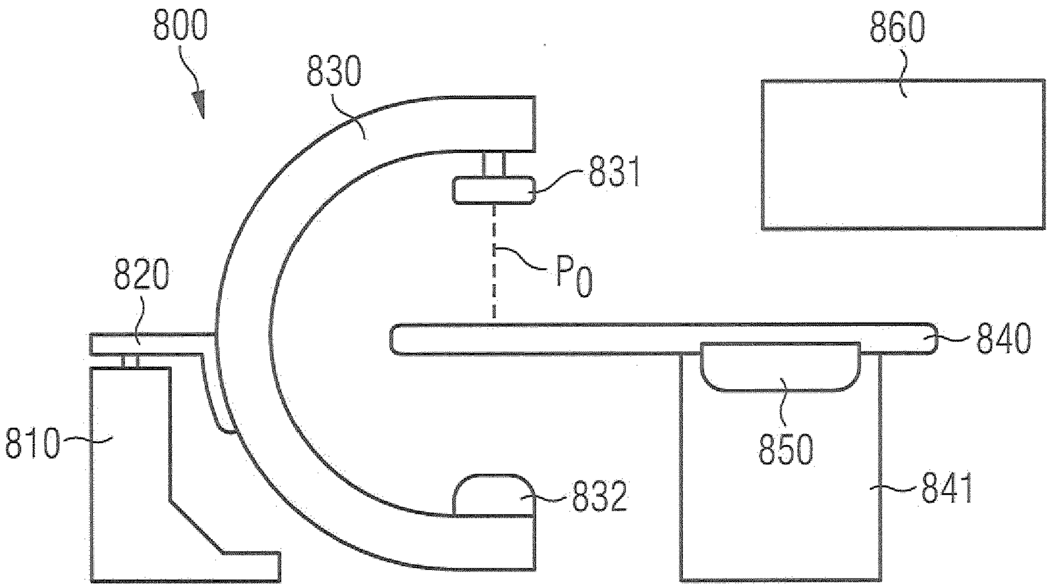

FIG 2

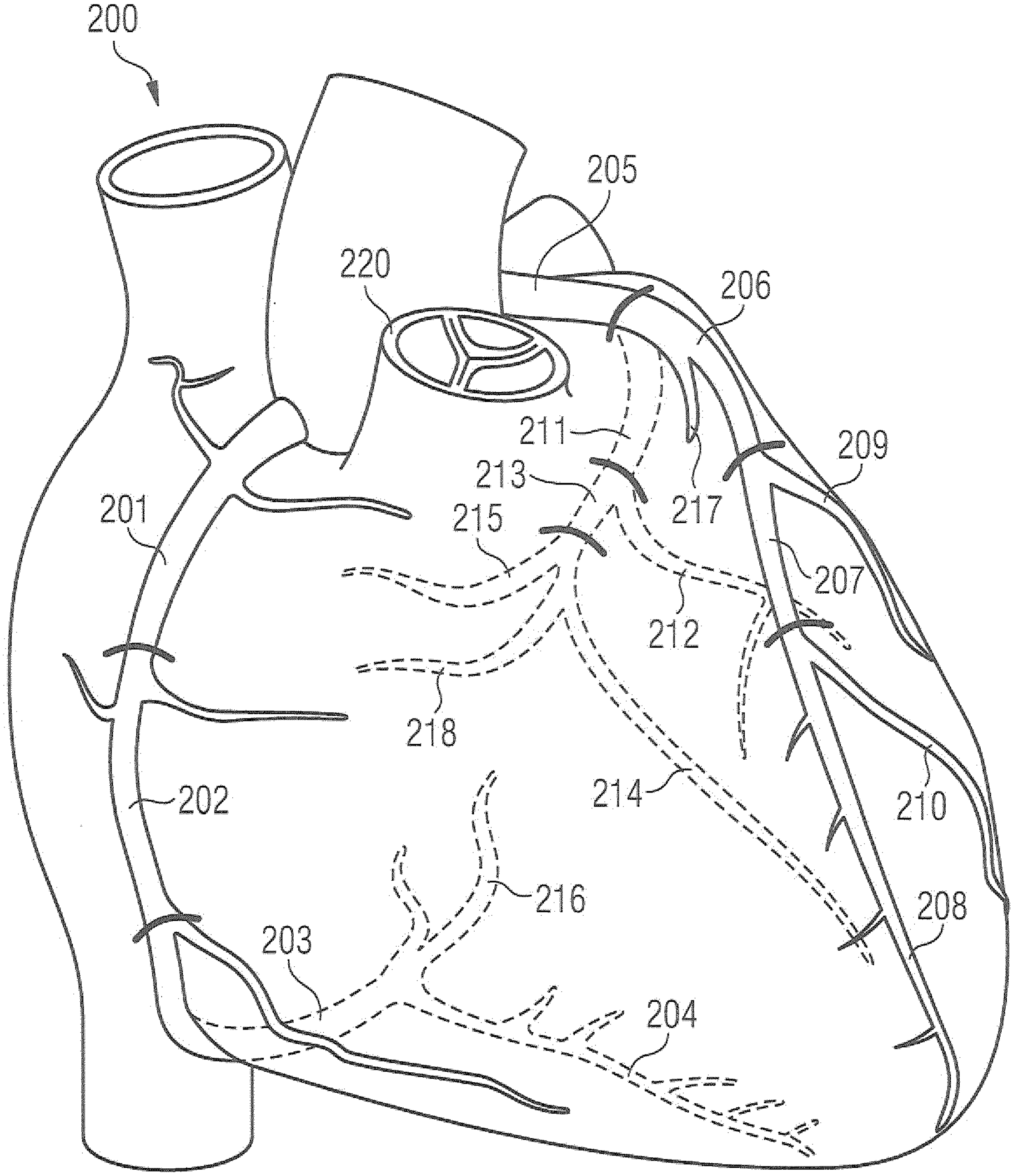
200
205
220
206
211
213
217
209
201
215
207
212
218
202
214
210
216
208
203
204

FIG 3

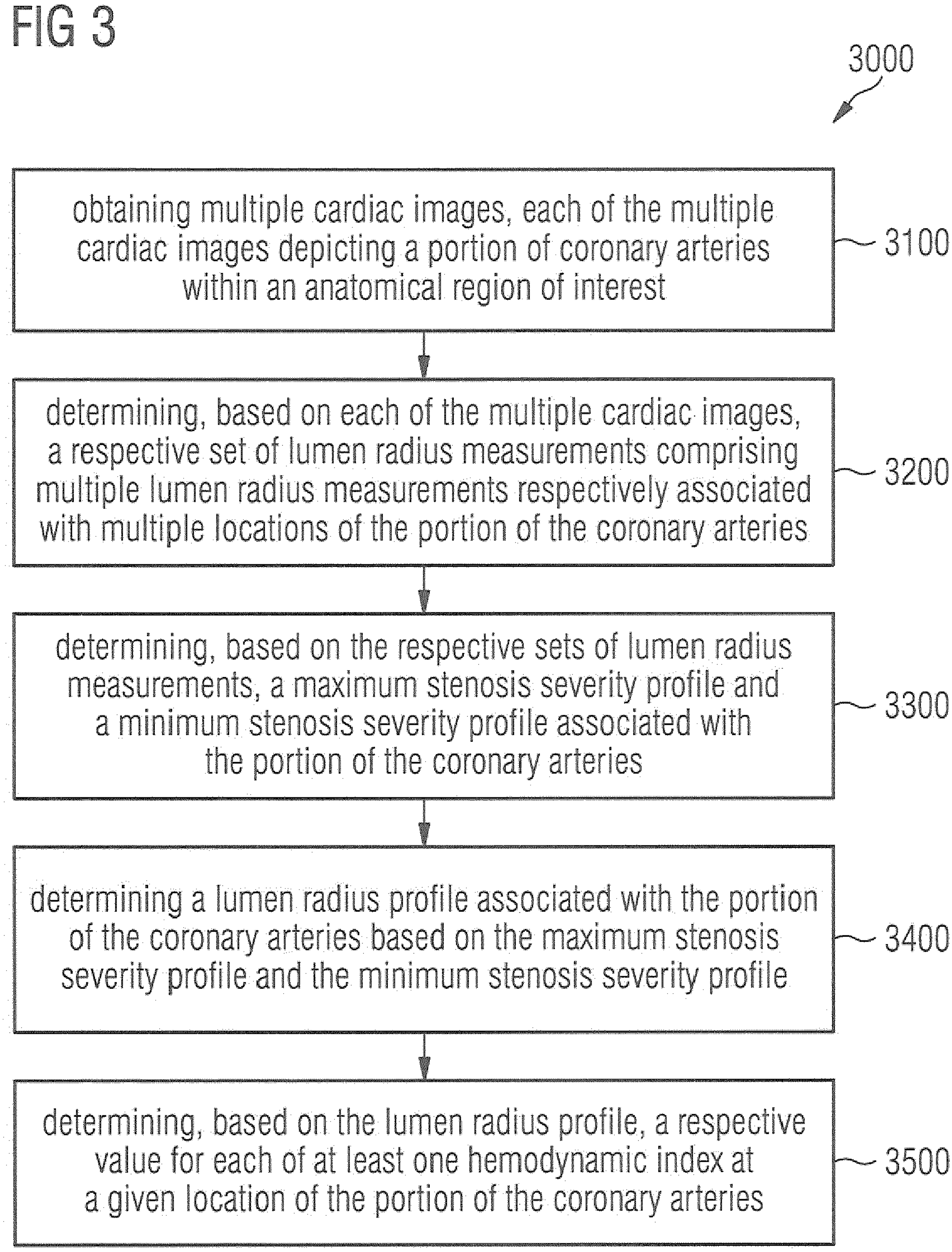
3000
obtaining multiple cardiac images, each of the multiple cardiac images depicting a portion of coronary arteries within an anatomical region of interest
3100
determining, based on each of the multiple cardiac images, a respective set of lumen radius measurements comprising multiple lumen radius measurements respectively associated with multiple locations of the portion of the coronary arteries
3200
determining, based on the respective sets of lumen radius measurements, a maximum stenosis severity profile and a minimum stenosis severity profile associated with the portion of the coronary arteries
3300
determining a lumen radius profile associated with the portion of the coronary arteries based on the maximum stenosis severity profile and the minimum stenosis severity profile
3400
determining, based on the lumen radius profile, a respective value for each of at least one hemodynamic index at a given location of the portion of the coronary arteries
3500

FIG 4

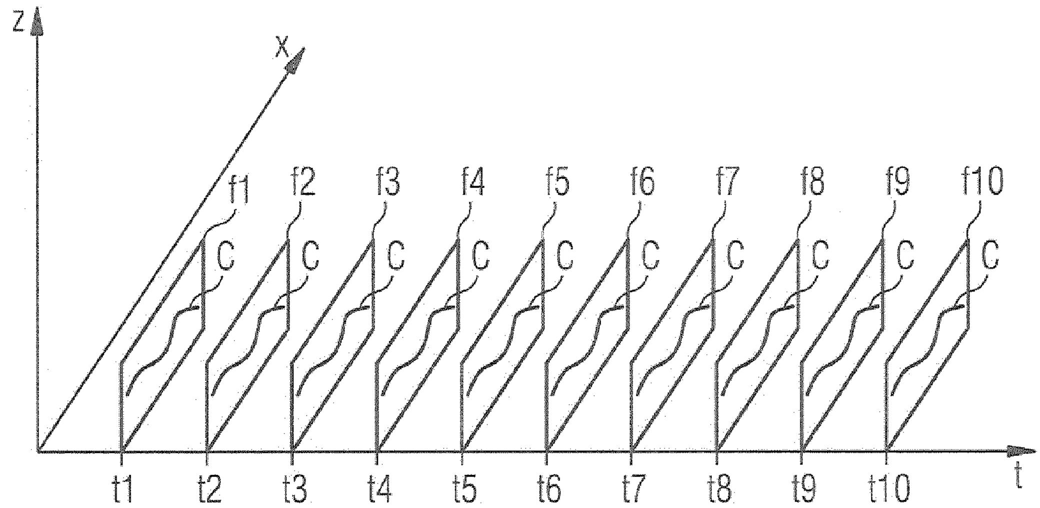
z
x
f1
f2
f3
f4
f5
f6
f7
f8
f9
f10
C
t1
t2
t3
t4
t5
t6
t7
t8
t9
t10
t

FIG 5

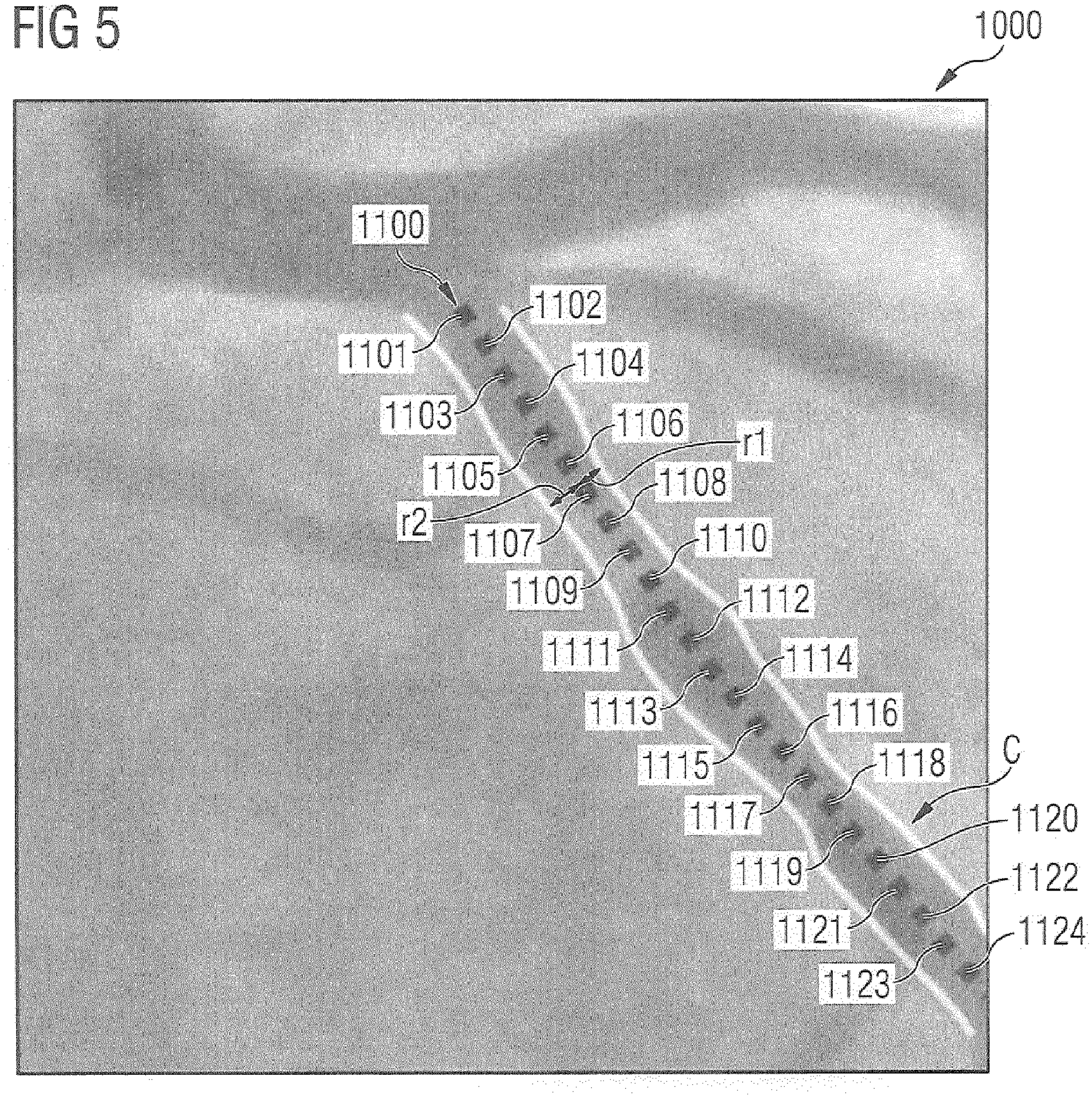
1000
1100
1101
1102
1103
1104
1105
1106
r1
r2
1107
1108
1109
1110
1111
1112
1113
1114
1115
1116
1117
1118
C
1119
1120
1121
1122
1123
1124

FIG 6

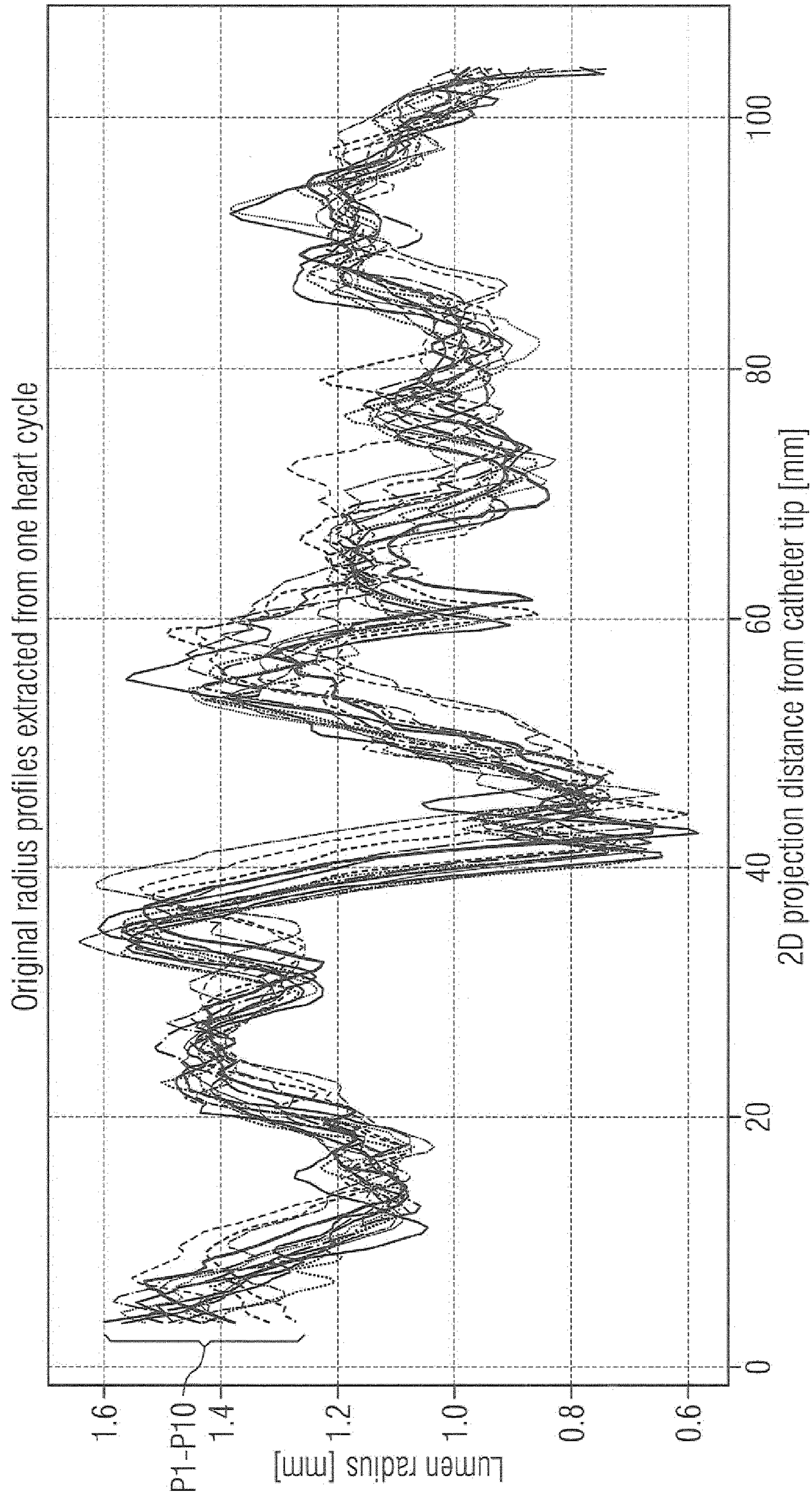
Original radius profiles extracted from one heart cycle
Lumen radius [mm]
1.6
1.4
1.2
1.0
0.8
0.6
P1–P10
0
20
40
60
80
100
2D projection distance from catheter tip [mm]

FIG 7

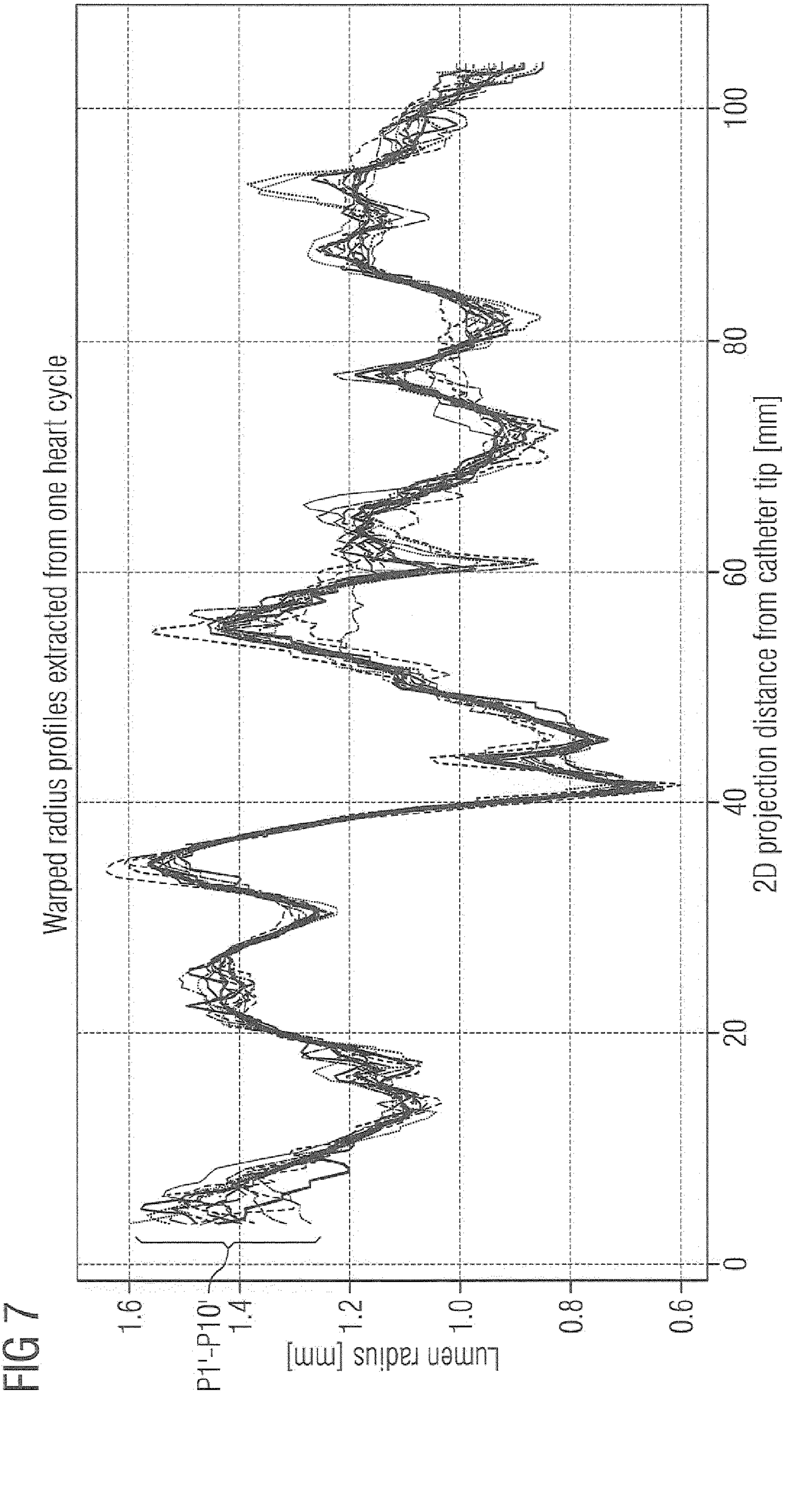

Warped radius profiles extracted from one heart cycle
Lumen radius [mm]
P1'-P10'
1.6
1.4
1.2
1.0
0.8
0.6
0
20
40
60
80
100
2D projection distance from catheter tip [mm]

FIG 8

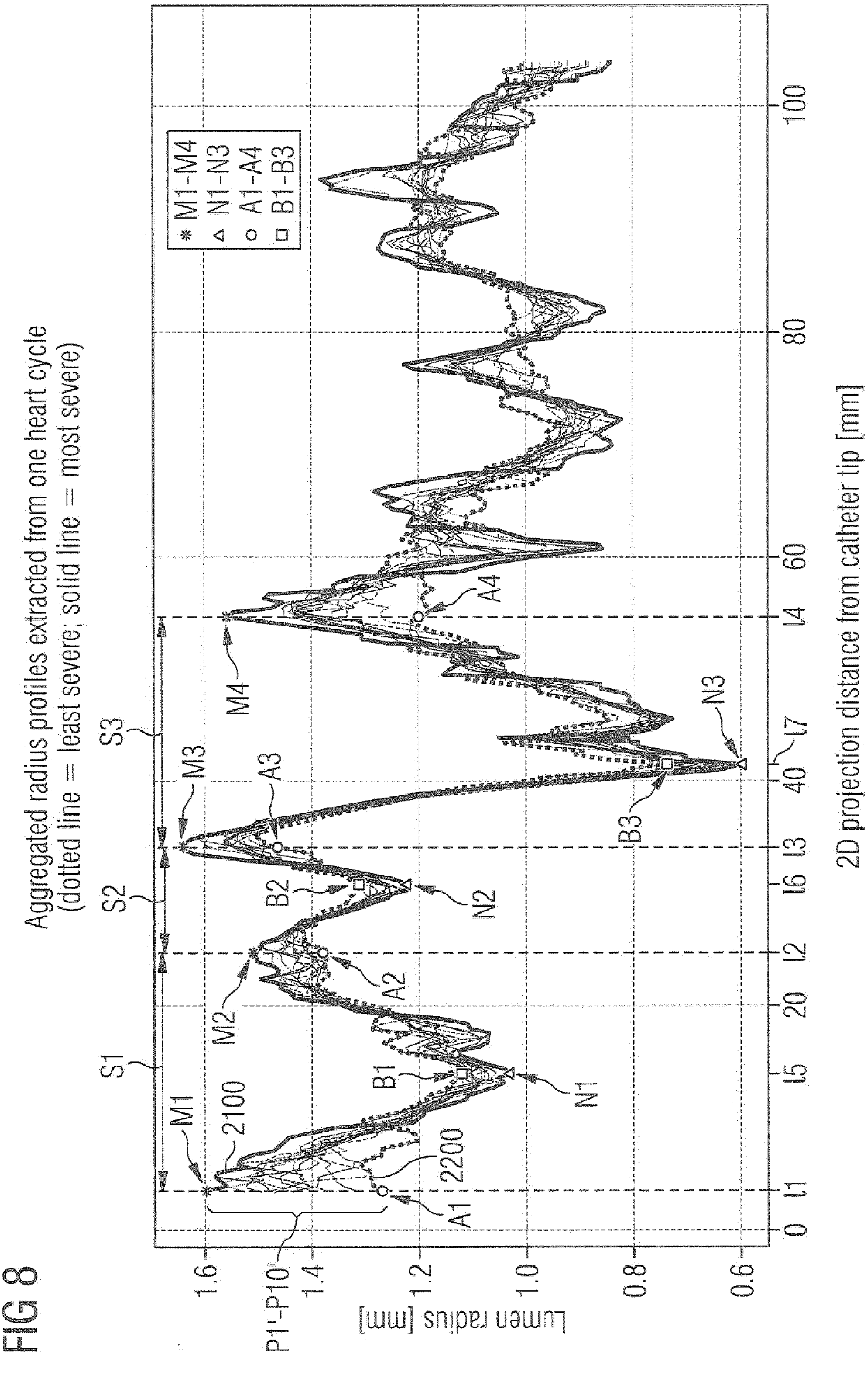
Aggregated radius profiles extracted from one heart cycle
(dotted line = least severe; solid line = most severe)
* M1-M4
△ N1-N3
○ A1-A4
□ B1-B3
Lumen radius [mm]
1.6
1.4
1.2
1.0
0.8
0.6
P1'-P10'
2D projection distance from catheter tip [mm]
0
l1
l5
20
l2
l6
l3
40
l7
l4
60
80
100
S1
S2
S3
M1
M2
M3
M4
N1
N2
N3
A1
A2
A3
A4
B1
B2
B3
2100
2200

FIG 9

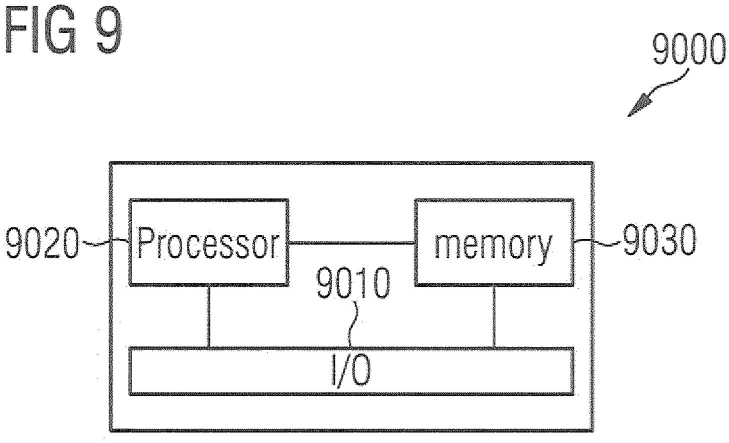

Europäisches Patentamt
European Patent Office
Office européen des brevets

# EUROPEAN SEARCH REPORT

Application Number
EP 23 46 5537

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X<br>Y<br>A | US 2018/344173 A1 (TU SHENGXIAN [CN] ET AL) 6 December 2018 (2018-12-06)<br>* In particular paragraphs 0029, 0041, 0071, 0036, 0043, 0056, 0057, 0046, 0058, 0059 * | 1,2,5,7, 8,10-17<br>6,9<br>3,4 | INV.<br>G06T7/00 |
| X<br>Y<br>A | US 2015/272448 A1 (FONTE TIMOTHY A [US] ET AL) 1 October 2015 (2015-10-01)<br>* In particular, paragraphs 0407, 0287,0164 * | 1,2,5,7, 8,10-17<br>6,9<br>3,4 | |
| X<br>A | US 2016/148371 A1 (ITU LUCIAN MIHAI [RO] ET AL) 26 May 2016 (2016-05-26)<br>* In particular, Figures 18, 24, and paragraphs 0090, 0092, 0102, 0110, 0127, 0138, 0140 * | 1,2,5-17<br>3,4 | |
| A | US 2021/298708 A1 (ABEN JEAN-PAUL [NL] ET AL) 30 September 2021 (2021-09-30)<br>* In particular paragraphs 0031, 0082-0086 * | 1-17 | TECHNICAL FIELDS SEARCHED (IPC)<br>G06T<br>G16H<br>A61B |

The present search report has been drawn up for all claims

1

| Place of search | Date of completion of the search | Examiner |
|---|---|---|
| The Hague | 22 February 2024 | Thean, Andrew |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

# ANNEX TO THE EUROPEAN SEARCH REPORT ON EUROPEAN PATENT APPLICATION NO. EP 23 46 5537

This annex lists the patent family members relating to the patent documents cited in the above-mentioned European search report. The members are as contained in the European Patent Office EDP file on
The European Patent Office is in no way liable for these particulars which are merely given for the purpose of information.

22-02-2024

| Patent document cited in search report | Publication date | Patent family member(s) | Publication date |
|---|---|---|---|
| US 2018344173 A1 | 06-12-2018 | CN 105326486 A | 17-02-2016 |
| | | DE 112016005603 T5 | 25-10-2018 |
| | | JP 6611959 B2 | 27-11-2019 |
| | | JP 6792768 B2 | 02-12-2020 |
| | | JP 2018535816 A | 06-12-2018 |
| | | JP 2019213903 A | 19-12-2019 |
| | | US 2018344173 A1 | 06-12-2018 |
| | | WO 2017097073 A1 | 15-06-2017 |
| US 2015272448 A1 | 01-10-2015 | EP 3127026 A1 | 08-02-2017 |
| | | JP 6378779 B2 | 22-08-2018 |
| | | JP 6634123 B2 | 22-01-2020 |
| | | JP 6905574 B2 | 21-07-2021 |
| | | JP 2017512577 A | 25-05-2017 |
| | | JP 2018196742 A | 13-12-2018 |
| | | JP 2020044375 A | 26-03-2020 |
| | | US 9087147 B1 | 21-07-2015 |
| | | US 2015272448 A1 | 01-10-2015 |
| | | US 2015324545 A1 | 12-11-2015 |
| | | US 2016180055 A1 | 23-06-2016 |
| | | US 2017220760 A1 | 03-08-2017 |
| | | US 2017329930 A1 | 16-11-2017 |
| | | US 2022406470 A1 | 22-12-2022 |
| | | WO 2015153362 A1 | 08-10-2015 |
| US 2016148371 A1 | 26-05-2016 | CN 106037710 A | 26-10-2016 |
| | | US 9349178 B1 | 24-05-2016 |
| | | US 2016148372 A1 | 26-05-2016 |
| | | US 2017245821 A1 | 31-08-2017 |
| | | US 2018153495 A1 | 07-06-2018 |
| | | US 2019038249 A1 | 07-02-2019 |
| | | US 2021219935 A1 | 22-07-2021 |
| US 2021298708 A1 | 30-09-2021 | EP 3884868 A1 | 29-09-2021 |
| | | JP 2021166701 A | 21-10-2021 |
| | | US 2021298708 A1 | 30-09-2021 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- **TRÖBS, MONIQUE et al.** Comparison of fractional flow reserve based on computational fluid dynamics modeling using coronary angiographic vessel morphology versus invasively measured fractional flow reserve. *The American journal of cardiology*, 2016, vol. 117 (1), 29-35
- **ITU, LUCIAN et al.** A machine-learning approach for computation of fractional flow reserve from coronary computed tomography. *Journal of applied physiology*, 2016, vol. 121 (1), 42-52
- **ASHBURNER, JOHN.** A fast diffeomorphic image registration algorithm. *Neuroimage*, 2007, vol. 38 (1), 95-113
- **HU, LIHUA et al.** KR-DBSCAN: A density-based clustering algorithm based on reverse nearest neighbor and influence space. *Expert Systems with Applications*, 2021, vol. 186, 115763
- **GARRONE, PAOLO et al.** Quantitative coronary angiography in the current era: principles and applications. *Journal of interventional cardiology*, 2009, vol. 22 (6), 527-536
- **SALVADOR, STAN** ; **PHILIP CHAN**. Toward accurate dynamic time warping in linear time and space. *Intelligent Data Analysis*, 2007, vol. 11 (5), 561-580
- Optimal subsequence bijection. **LATECKI, LONGIN JAN et al.** Seventh IEEE International Conference on Data Mining (ICDM 2007). IEEE, 2007
- **SANKARAN, SETHURAMAN et al.** Uncertainty quantification in coronary blood flow simulations: impact of geometry, boundary conditions and blood viscosity. *Journal of biomechanics*, 2016, vol. 49 (12), 2540-2547